# EUROPEAN PATENT APPLICATION

(11) **EP 1 327 624 A1**
(43) Date of publication of application: **16.07.2003**
(21) Application number: 01974857.3
(22) Date of filing: 15.10.2001
(51) Int. Cl.: C07C 67/52, C07C 67/62, C07C 69/33, C12P 7/64

(54) **METHOD OF PURIFYING PRAVASTATIN OR ITS PHARMACOLOGICALLY ACCEPTABLE SALT**

(30) Priority: 16.10.2000 JP 2000315255
(71) Applicant: Sankyo Company, Limited, Tokyo 103-8426 (JP)
(72) Inventor: SUGIO, Nobunari, c/o SANKYO COMPANY, LIMITED, Iwaki-shi, Fukushima 971-8183 (JP); KOJIMA, Shunshi, c/o SANKYO COMPANY, LIMITED, Hiratsuka-shi, Kanagawa 254-0014 (JP); SUZUKI, Mutsuo, c/o SANKYO COMPANY, LIMITED, Hiratsuka-shi, Kanagawa 254-001 4 (JP); HAMANO, Kiyoshi, c/o SANKYO COMPANY, LIMITED, Shinagawa-ku, Tokyo 140-8710 (JP); TAKAMATSU, Yasuyuki, c/o SANKYO COMPANY, LIMITED, Iwaki-shi, Fukushima 971-8183 (JP); HAGISAWA, Minoru, c/o SANKYO COMPANY, LIMITED, Hiratsuka-shi, Kanagawa 254-0014 (JP)
(74) Representative: Gibson, Christian John Robert
(86) International application number: JP0109044
(87) International publication number: WO02032847

(57) **Abstract**

The present invention provides methods for purification of pravastatin or a pharmacologically acceptable salt thereof using a salting-out technique.

## Description

### [Technical field of the invention]

The present invention relates to a method for purification of pravastatin or a pharmacologically acceptable salt thereof, the method comprising use of a salting-out technique.

### [Background of the invention]

Pravastatin is disclosed in the specification of Japanese Patent Application Publication No. Sho 57-2240 (USP 4346227) as an HMG-CoA reductase inhibitor and is the compound of formula (I). Pravastatin sodium has been placed on the market as an anti-hyperlipidemic agent.

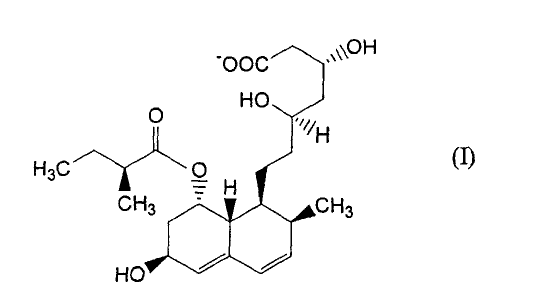

The following methods for purification of HMG-CoA reductase inhibitors, including pravastatin, are known.
(1) A method for purification of HMG-CoA reductase inhibitors by high performance liquid chromatography is disclosed in the specification of WO 92/16276 (Japanese Patent Application Publication (Kohyo) No. Hei 6-506210). The HMG-CoA reductase inhibitors disclosed therein, such as fat-soluble lovastatin and simvastatin, are in lactone form. Pravastatin sodium is inherently different from lactone form, fat-soluble HMG-CoA reductase inhibitors. A method for purification of HMG-CoA reductase inhibitors by displacement chromatography is disclosed in the specification of WO 00/17182. These methods for chromatographic purification, such as by high performance liquid chromatography and displacement chromatography, are complicated and impractical in view of the industrial production of HMG-CoA reductase inhibitors.
(2) A method for isolation or purification of an HMG-CoA reductase inhibitor which comprises adjusting the pH of a concentrated cultured broth, containing HMG-CoA reductase inhibitor, to the range from 4.5 to 7.5 with an acid, extracting the HMG-CoA reductase inhibitor with ethyl acetate, if necessary, lactonization of the inhibitor and crystallization to give the HMG-CoA reductase inhibitor having 99.6% or more purity, is disclosed in the specification of WO 99/42601. A method for isolation or purification of pravastatin is described in Example 3 of this specification. In this example, the purity of pravastatin obtained from the extract with ethyl acetate is only 70.3%. This crude product is purified by chromatography to afford the desired product; however, the purity of this product is not disclosed. It is not clear that the final product is highly pure pravastatin.

In the example of this specification, it is reported that lovastatin with a purity of 99.6% or higher was obtained. However the HPLC chart (Fig 4) of the final product in this specification calls this purity figure into question.

Clearly, difficult chromatography techniques are required in the prior art in order to purify pravastatin.

The methods for purification according to this invention comprise salting-out techniques. The term 'salting-out technique' refers to a method for precipitating a solute from an aqueous solution by the addition of an inorganic salt. Salting-out techniques are usually used in the purification of macromolecular compounds such as proteins and amino acids. For example, when a large amount of a salt is added to an aqueous solution of protein molecules, interaction of protein molecules and water molecules is prevented, resulting in precipitation of the protein molecules.
On the other hand, when an inorganic salt is added to an aqueous solution of a low molecular weight compound, salt crystals precipitate in certain cases. This case is different from the salting-out of a macromolecular compound described hereinabove. In this case, when a salt is added to a solution, molecules of water around the molecule of the salt are fixed by hydrating ability of the salt to form hydrates; consequently, the number of molecules of water available to dissolve the solute decreases, resulting in precipitation of the solute. It has been considered that it is difficult to selectively precipitate a particular solute with high purity from an aqueous solution containing many compounds having very similar chemical structures. For this reason, there has been no disclosure and no suggestion of a method for purification of pravastatin and other HMG-CoA reductase inhibitors by a salting-out techinique.

Many HMG-CoA reductase inhibitors other than pravastatin are known, for example atorvastatin, fluvastatin and itavastatin all of which are synthetically prepared. On the other hand lovastatin and simvastatin are prepared by fermentation as well as pravastatin. These compounds are obtained by a one-step fermentation process; however, pravastatin is obtained by a two-step fermentation process. According to the following reaction scheme pravastatin sodium is produced by the first fermentation step followed by microbial conversion of the product of the first step by the second fermentation.

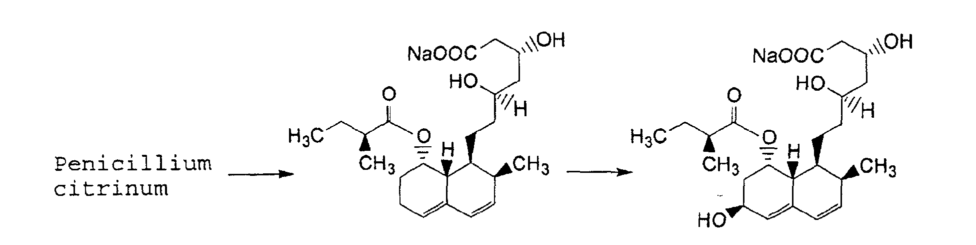

In general, many unexpected impurities are obtained by a fermentation process compared with a chemical synthesis. When the products of each fermentation process are purified, all of the impurities cannot be separated from the final product. It is further difficult to remove the impurities from the product in an industrial-scale fermentation process.

On the other hand there is a decision (Tokyo High Court decision No. Hei 9 (gyou ke) 302; date of decision February 17, 2000) which described that one of the important factors in the preparation of a safe and effective pharmaceutical agent is to obtain a product with high purity; a chemical substance can be produced by chemical synthesis from one or more starting materials, by fermentation of microorganism or by using the cells prepared by genetic recombination, followed by isolation or purification of the product; in many cases, it is difficult to obtain the chemical substance with 100% purity by any one of the processes including genetic recombination process, because the purity of the chemical substance depends on the purity of the starting material(s), versatility of the reaction and decomposition reaction during isolation or purification process; and that in the viewpoint of those skilled in the pharmaceutical field, the possibility of adverse effect of impurities in a chemical substance on treatment and diagnosis of a disease is indisputable; it is therefore important to obtain a chemical substance with as high a purity as possible.
It is particularly necessary for HMG-CoA reductase inhibitors to be as pure as possible to avoid side effects, because they are administered on a long term basis in order to effectively lower the level of cholesterol in the blood.
The purification process of pravastatin is important because pravastatin produced through a two-step fermentation process contains more impurities than simvastatin and lovastatin produced through a one-step fermentation process. It is therefore desirable to provide a process for isolating and purifying pravastatin or a pharmacologically acceptable salt thereof to obtain a product, the purity of which is as the same as that obtained by chromatography, while maintaining good industrial productivity and avoiding impractical and industrially disadvantagerous methods such as chromatography.

### [Disclosure of the invention]

The inventors have made a great effort for many years to study processes for isolating and purifying pravastatin and pharmacologically acceptable salts thereof in a purity equivalent to that obtained by chromatography while maintaining industrial productivity. They have found that pravastatin, which has a low molecular weight, is selectively precipitated in a high purity by a salting-out technique and completed this invention.

The present invention relates to:
(1) a method for purification of pravastatin or a pharmacologically acceptable salt thereof using a salting-out technique;
   preferably
(2) a method according to (1) wherein the salt employed in the salting-out technique is an alkali metal salt, an alkaline earth metal salt or an ammonium salt;
(3) a method according to (1) wherein the salt employed in the salting-out technique is an alkali metal salt or an ammonium salt;
(4) a method according to (1) wherein the salt employed in the salting-out technique is sodium chloride, ammonium sulfate, ammonium acetate or ammonium nitrate;
(5) a method according to (1) wherein the salt employed in the salting-out technique is sodium chloride;
(6) a method according to any one of (1) to (5) further comprising decomposing the impurities using an inorganic base;
(7) a method according to any one of (1) to (6) further comprising decomposing the impurities using an inorganic acid;
(8) a method according to (7) wherein the inorganic acid is phosphoric acid or sulfuric acid,
(9) a method according to (7) wherein the inorganic acid is phosphoric acid,
(10) a method according to any one of (7) to (9) wherein the pH of the solution in the inorganic acid decomposition process is in the range from 2 to 5;
(11) a method for purification of pravastatin sodium according to any method selected from (1) to (10).

Of the possible salts employed in the salting-out technique of this invention,
the alkali metal salt is a salt containing an alkali metal, for example, an alkali metal halide such as lithium chloride, potassium chloride or sodium chloride; an alkali metal carboxylic acid salt such as lithium acetate, potassium acetate, sodium acetate, lithium formate, potassium formate or sodium formate; or an alkali metal inorganic acid salt such as lithium nitrate, potassium nitrate, sodium nitrate, lithium sulfate, potassium sulfate or sodium sulfate.
The alkaline earth metal salt is a salt containing an alkaline earth metal, for example, an alkaline earth metal halide such as magnesium chloride; an alkaline earth metal carboxylic acid salt such as magnesium acetate or magnesium formate; or an alkaline earth metal inorganic acid salt such as magnesium nitrate or magnesium sulfate.
The ammonium salt is, for example, an ammonium salt such as ammonium chloride, ammonium acetate, ammonium formate, ammonium nitrate or ammonium sulfate.

In addition, the salt employed in the salting-out technique of this invention is not particularly limited provided that it is usually used as a salt, is preferably an alkali metal salt, an alkaline earth metal salt or an ammonium salt; more preferably an alkali metal salt or an ammonium salt; still more preferably sodium chloride, ammonium sulfate, ammonium acetate or ammonium nitrate and most preferably sodium chloride.
The pharmacologically acceptable salt in the definition of 'pravastatin or a pharmacologically acceptable salt thereof' is not particularly limited provided that it has no pharmacological side-effects and can usually be used as a salt, is, for example, an alkali metal salt such as a sodium salt, a potassium salt or a lithium salt; an alkaline earth metal salt such as a calcium salt or a magnesium salt; a metal salt such as an aluminum salt or an iron salt; an inorganic salt such as an ammonium salt; a metal salt such as an aluminum salt, an iron salt, a zinc salt, a copper salt, a nickel salt, or a cobalt salt; an amine salt, for example, an organic salt such as a t-octylamine salt, a dibenzylamine salt, a morpholine salt, a glucosamine salt, a phenylglycine alkyl ester salt, an ethylenediamine salt, an N-methylglucamine salt, a guanidine salt, a diethylamine salt, a triethylamine salt, a dicyclohexylamine salt, an N,N'-dibenzylethylenediamine salt, a chloroprocaine salt, a procaine salt, a diethanolamine salt, an N-benzylphenethylamine salt, a piperazine salt, a tetramethylammonium salt or a tris(hydroxymethyl)aminomethane salt; or an amino acid salt such as a glycine salt, a lysine salt, a alginine salt, an ornithine salt, a glutamic acid salt or an aspartic acid salt; preferably an alkali metal salt, an alkaline earth metal salt, an inorganic salt or a metal salt; more preferably an alkali metal salt and most preferably a sodium salt.
The purification process of this invention can be accomplished as follows.
The salting-out technique of this invention is carried out by addition of an inorganic salt to a solution or suspension of pravastatin or a pharmacologically acceptable salt thereof in water; cooling the mixture; addition of seed crystals of pravastatin to the cooled mixture; followed by re-cooling the resulting mixture to afford a precipitate of pravastatin salt.
The salt employed in the salting-out technique may or may not have the same cation as that of the pharmacologically acceptable salt of pravastatin.

The amount of inorganic salt, which is added to the solution or suspension of pravastatin or a pharmacologically acceptable salt thereof, is preferably from 5% to 40% of the amount of the water mentioned above and most preferably from 15% to 35%.

After addition of the inorganic salt to the solution or suspension of pravastatin or pharmacologically acceptable salt thereof, the temperature to which the resulting mixture is heated varies depending on the inorganic salt employed, and is usually from 20°C to 60°C and preferably from 30°C to 45°C.

The method for purification of pravastatin or a pharmacologically acceptable salt thereof comprises a salting-out technique and optionally further decomposition of impurities by an inorganic base and/or further decomposition of impurities by an inorganic acid. Each process is conducted as follows.

The inorganic base employed in the decomposition of impurities by an inorganic base is not particularly limited provided that it can usually be used as an inorganic base in reactions, and is, for example, an alkali metal carbonate such as lithium carbonate, sodium carbonate or potassium carbonate; an alkali metal hydrogencarbonate such as lithium hydrogencarbonate, sodium hydrogencarbonate or potassium hydrogencarbonate; an alkali metal hydride such as lithium hydride, sodium hydride or potassium hydride; an alkali metal hydroxide such as lithium hydoxide, sodium hydroxide or potassium hydroxide; or an alkali metal alkoxide such as lithium methoxide, sodium methoxide, sodium ethoxide or potassium t-butoxide; preferably an alkali metal hydroxide and most preferably sodium hydroxide.

The decomposition of impurities present with pravastatin or a pharmacologically acceptable salt thereof by an inorganic base is carried out in the presence or absence of an inert solvent (preferably in the presence of a solvent) at a pH of from 10 to 14.
The reaction temperature and reaction time for the decomposition of impurities present with pravastatin or a pharmacologically acceptable salt thereof by an inorganic base depends on the pH: essentially, when the reaction temperature is low, the reaction time is long; when the reaction temperature is high, the reaction time is short. For example, the reaction temperature is from -10°C to 110°C and the reaction time from 15 minutes to 200 hours.

The base-decomposition (using an inorganic base) of impurities present in the concentrated cultured broth containing pravastatin produced by a microorganism is preferably conducted at a pH of from 11 to 14 (more preferably from 11 to 12), at a reaction temperature of from 40°C to 110°C (more preferably from 95°C to 105°C) and for a reaction time in the range of from 2 to 24 hours (more preferably from 2 to 5 hours).
On the other hand the base-decomposition process (using an inorganic acid) of impurities contained in the reverse extract obtained by extraction with an aqueous alkaline solution (preferably at a pH of from 8 to 9) from the organic extract solution which is obtained by extraction of a concentrated cultured broth containing pravastatin produced by a microorganism with an organic solvent under acidic conditions (preferably at a pH of from 4 to 6), is preferably conducted at a pH of from 13 to 14 (more preferably from 13.5 to 14), at a reaction temperature of from -10°C to 50°C (more preferably from -5°C to 5°C) and for a reaction time of from 2 to 180 hours (more preferably from 20 to 50 hours and most preferably from 25 to 35 hours).

The inert solvent employed in the base-decomposition process (using an inorganic base) of impurities present with pravastatin or a pharmacologically acceptable salt thereof is not particularly limited provided that it has no adverse effect on the reaction and can usually be used as a solvent and is, for example, an alcohol such as methanol, ethanol, n-propanol, iso-propanol, n-butanol, iso-butanol, t-butanol, isoamyl alcohol, di(ethylene glycol), glycerol, octanol, cyclohexanol or methyl cellosolve; water; or a mixture of water and any of the alcohols mentioned above; preferably water or a mixture of water and any of the alcohols mentioned above; and most preferably water or a mixture of water and ethanol.

After the reaction the desired product, pravastatin, is isolated from the reaction mixture according to a conventional procedure. For example, it can be obtained by addition of an aqueous acid solution such as aqueous sulfuric acid to the reaction mixture; extraction of the resulting mixture with an organic solvent immiscible with water, such as ethyl acetate; washing the organic layer containing the desired product with water; followed by evaporation of the solvent. A pravastatin salt can be obtained by treatment of the organic layer, if necessary, with activated charcoal in order to decolorize, removal of the activated charcoal by filtration, addition of a salt-forming agent such as sodium methoxide, sodium ethoxide or sodium hydroxide to the filtrate, followed by concentration of the resulting mixture under reduced pressure by a rotary evaporator or the like.

The inorganic acid employed in the decomposition of impurities using an inorganic acid is not particularly limited provided that it can usually be used as an inorganic acid and is, for example, an inorganic acid such as hydrobromic acid, hydrochloric acid, sulfuric acid, perchloric acid, phosphoric acid or nitric acid; preferably phosphoric acid or sulfuric acid and most preferably phosphoric acid.

The decomposition process of impurities present with pravastatin or a pharmacologically acceptable salt thereof by an inorganic acid is carried out in the presence or absence of an inert solvent (preferably in the presence of a solvent) at a pH of from 2 to 5 (preferably at a pH of from 3 to 4).

The reaction temperature and reaction time for the decomposition process of impurities present with pravastatin or a pharmacologically acceptable salt thereof by an inorganic acid depends on the pH: essentially, when the reaction temperature is low, the reaction time is long; when the reaction temperature is high, the reaction time is short. For example, the reaction temperature is from 20°C to 80°C (preferably from 40°C to 60°C) and the reaction time is from 1 minute to 6 hours (preferably from 5 to 20 minutes).

The inert solvent employed in the acidic decomposition process (using an inorganic acid) of impurities present with pravastatin or a pharmacologically acceptable salt thereof is not particularly limited provided that it has no adverse effect on the reaction and includes the same solvents as those employed in the basic decomposition process (using an inorganic base) of the impurities mentioned above.
After the reaction, the desired product, pravastatin, is isolated from the reaction mixture according to a conventional procedure. For example it can be obtained by extraction of the reaction mixture with an organic solvent immiscible with water, such as ethyl acetate; washing the organic layer containing the desired product with water; followed by evaporation of the solvent. A pravastatin salt can be obtained by, if necessary, treatment of the organic layer with activated charcoal in order to decolorize, removal of the activated charcoal by filtration, addition of a salt-forming agent such as sodium hydroxide, sodium methoxide or sodium ethoxide to the filtrate, followed by concentration of the resulting mixture under reduced pressure by a rotary evaporator or the like.

In addition, if necessary, the purified pravastatin or pharmacologically acceptable salt obtained above can also be crystallized according to a conventional procedure well known to those skilled in the art of organic synthesis (for example Ullmann's, Encyclopedia of Industrial Chemistry, Vol. A24, 5th edition (1993) pp 435-505) as follows.
A crystalline form of pravastatin or a pharmacologically acceptable salt thereof can be obtained by dissolving the purified pravastatin or pharmacologically acceptable salt thereof obtained according to the procedure mentioned above in an organic solvent or water under heating, followed by seeding.
The organic solvent employed in the crystallization is, for example, an aliphatic hydrocarbon such as hexane or heptane; an aromatic hydrocarbon such as toluene or xylene; an ester such as methyl acetate, ethyl acetate, propyl acetate, butyl acetate or diethyl carbonate; an organic acid such as acetic acid; an alcohol such as methanol, ethanol, n-propanol, iso-propanol, n-butanol, iso-butanol, t-butanol, isoamyl alcohol, di(ethylene glycol), glycerol or octanol; a ketone such as acetone or methyl ethyl ketone; an ether such as diethyl ether, diisopropyl ether, tetrahydrofuran, dioxane, dimethoxyethane, di(ethylene glycol) dimethyl ether; an amide such as formamide, dimethylformamide, dimethylacetamide or hexamethylphosphoric triamide; a sulfoxide such as dimethyl sulfoxide; or a mixture of water and one or more of the organic solvents mentioned above, preferably a mixture of water and one or more of the organic solvents selected from the group consisting of alcohols, esters and ketones, and most preferably a mixture of water, an alcohol and an ester.

### [Advantage of the invention]

Pravastatin or a pharmacologically acceptable salt thereof with high purity can be obtained by the purification process of this invention without using chromatography, which is inefficient when carried out on an industrial scale and is impractical and unproductive.

### [Possibility of industrial application]

When pravastatin or a pharmacologically acceptable salt thereof of this invention is used as a pharmaceutical agent, it can be administered alone; orally administered in a unit dosage form such as a tablet, capsule, granule, powder or syrup prepared by mixing it with pharmacologically acceptable appropriate excipient(s), diluent(s) and the like; or parenterally administered in a unit dosage form such as injection, suppository or the like.

The purity of the product of the present invention can be determined by high performance liquid chromatography (HPLC). The conditions of the HPLC are as follows:
A: Mobile phase: a mixture of methanol:water:glacial acetic acid:triethylamine (600:400:1:1);
   Wavelength of detection: UV 238 nm;
   Column: ERC-ODS-1262 φ6mm×10cm (product of Elmer Optics Co., Ltd.);
   Column temperature: 30°C;
   Flow rate: 1 ml/min.
B: Mobile phase: a mixture of methanol:water:glacial acetic acid:triethylamine = 450:550:1:1;
   Wavelength of detection: UV 238 nm;
   Column: Ultrasphere ODS 5µm, φ 4.6 mm × 15 cm (product of Beckmann Co., Ltd.);
   Column temperature: 25°C;
   Flow rate: 1.3 ml/min,
   or
C: Mobile phase: 20% acetonitrile, 30% methanol, 50% TEAP buffer solution (0.3% triethylamine-H₃PO₄ (pH 3.2));
   Wavelength of detection: UV 238 nm;
   Column: Symmetry C18 3.5 µm, 4.6 mm × 15 cm (product of Waters Co.,Ltd.);
   Flow rate: 1 ml/min.

The present invention will be further illustrated by Reference examples and Examples, However, the scope of this invention is not limited to or by these Examples.

### Reference example 1

Ethyl acetate (264 ml) was added to a concentrated cultured broth (200 ml) containing 21 g of pravastatin sodium. The resulting mixture was adjusted to pH 5.4 with 20% aqueous sulfuric acid solution with stirring at room temperature. The ethyl acetate layer (1) was separated from the aqueous layer (1) of the resulting mixture. The aqueous layer (1) was extracted with ethyl acetate (264 ml) to give the ethyl acetate layer (2). The ethyl acetate layers (1) and (2) were combined and water (100 ml) was added to the ethyl acetate layers. The resulting mixture was adjusted to pH 8.7 with 48% aqueous sodium hydroxide solution with stirring at room temperature. The ethyl acetate layer (3) was separated from the aqueous layer (3) of the resulting mixture. The aqueous layer (3) was placed into a 500 ml round flask and concentrated under reduced pressure until the volume become 2/5 to afford an aqueous concentrated reverse extract (88 ml containing 19.3 g pravastatin sodium).

### Example 1

The aqueous concentrated reverse extract (3 ml) obtained from Reference example 1 (the extract contained 78.69% pure pravastatin sodium, the purity of which was determined by HPLC under the conditions referred to above as A) was placed into a test tube. This sample was heated to about 50°C, sodium chloride (0.90 g) was added thereto and then crystalline seeds of pravastatin sodium were added at 33°C thereto. The resulting mixture was cooled to 0°C, filtered and the crystals were washed with cold water to afford crystals of pravastatin sodium obtained by salting-out technique. The crystals contained 91.36 % pure pravastatin sodium, the purity of which was determined by HPLC under the conditions referred to above as A.

### Example 2

The aqueous concentrated reverse extract (3 ml) obtained from Reference example 1 (the extract contained 78.69% pure pravastatin sodium, the purity of which was determined by HPLC under the conditions referred to above as A) was placed into a test tube. This sample was heated to about 50°C, ammonium sulfate (0.83 g) was added thereto (oily products separated into an upper layer and a lower layer) and then crystalline seeds of pravastatin sodium were added thereto at 33°C. The resulting mixture was cooled to 0°C, filtered and the crystals were washed with cold water to afford crystals of pravastatin sodium obtained by salting-out technique (the upper layer crystals comprised a brown solid and the lower layer crystals comprised a white slurry solid). The upper layer crystals contained 83.82 % pure pravastatin sodium and the lower layer crystals contained 91.74% pure pravastatin sodium, the purities of which were determined by HPLC under the conditions referred to above as A.

### Example 3

The aqueous concentrated reverse extract (3 ml) obtained from Reference example 1 (the extract contained 78.69% pure pravastatin sodium, the purity of which was determined by HPLC under the conditions referred to as A) was placed into a test tube. This sample was heated to about 50°C, ammonium acetate (0.83 g) was added thereto and crystals precipitated a few minutes after addition. The resulting mixture was cooled to 0°C, cold water (2 ml) was added thereto and the mixture was slurried and filtered. The crystals were washed with cold water to afford crystals of pravastatin sodium obtained by salting-out technique. The crystals contained 90.10% pure pravastatin sodium, the purity of which was determined by HPLC under the conditions referred to as A.

### Example 4

The aqueous concentrated reverse extract (3 ml) obtained from Reference example 1 (the extract contained 78.69% pure pravastatin sodium, the purity of which was determined by HPLC under the conditions referred to as A) was placed into a test tube. This sample was heated to about 50°C, ammonium acetate (0.83 g) was added thereto and crystals precipitated a few minutes after addition. Water (3 ml) was added to the resulting mixture. The mixture was heated to about 50°C and the crystals were dissolved. Crystalline seeds of pravastatin sodium were added to the resulting mixture at 33°C and the mixture was cooled to 0°C. The crystals were collected by filtration and washed with cold water to afford crystals of pravastatin sodium obtained by salting-out technique. The crystals contained 93.22% pure pravastatin sodium, the purity of which was determined by HPLC under the conditions referred to above as A.

### Example 5

The aqueous concentrated reverse extract (3 ml) obtained from Reference example 1 (the extract contained 78.69% pure pravastatin sodium, the purity of which was determined by HPLC under the conditions referred to above as A) was placed into a test tube. This sample was heated to about 50°C, ammonium nitrate (0.83 g) was added thereto and then crystalline seeds of pravastatin sodium were added thereto at 33°C. The resulting mixture was cooled to 0°C, filtered and the crystals were washed with cold water to afford crystals of pravastatin sodium obtained by salting-out technique. The crystals contained 93.18% pure pravastatin sodium, the purity of which was determined by HPLC under the conditions referred to above as A.

### Example 6

The aqueous concentrated reverse extract (3 ml) obtained from Reference example 1 (the extract contained 78.69% pure pravastatin sodium, the purity of which was determined by HPLC under the conditions referred to above as A) was placed into a test tube. This sample was heated to about 50°C, ammonium nitrate (0.83 g) was added thereto and, when the mixture was cooled to 38°C, crystals precipitated. This mixture was heated to 53°C; however, the crystals were not dissolved. The resulting mixture was cooled to 0°C, filtered and the crystals were washed with cold water to afford crystals of pravastatin sodium obtained by salting-out technique. The crystals contained 93.37% pure pravastatin sodium, the purity of which was determined by HPLC under the conditions referred to above as A.

### Example 7

### (1) Purification by base-decomposition of impurities (using an inorganic base)

A concentrated cultured broth (300 ml) containing 37 g of pravastatin sodium was placed in a four-neck round-bottom flask (500 ml), heated to 100°C and 2 equivalents of aqueous sodium hydroxide in aqueous solution was added thereto (pH 11.5). The mixture was stirred at 100°C for 3 hours, cooled to room temperature and then the mixture was adjusted to pH 9.0 with 20% aqueous sulfuric acid solution at room temperature to give an alkaline solution containing 33 g of pravastatin sodium. Ethyl acetate (132 ml) was added to this alkaline solution (70 ml) containing 5.3 g of pravastatin sodium and the mixture was adjusted to pH 5.4 with 20% aqueous sulfuric acid solution at room temperature with stirring. The ethyl acetate layer (1) was separated from the aqueous layer (1) of the mixture. The aqueous layer (1) was extracted with ethyl acetate (132 ml) to give the ethyl acetate layer (2). On the other hand the ethyl acetate layer (1) was washed with water (25 ml) to give the ethyl acetate layer (3) and aqueous layer (3). The ethyl acetate layer (2) was washed with aqueous layer (3) to give the ethyl acetate layer (4). Water (60 ml) was added to the ethyl acetate layer (3) and the pH of the mixture was adjusted to 8.7 with 48% aqueous sodium hydroxide solution at room temperature with stirring. The aqueous layer (5) was separated from the ethyl acetate layer. The pH of a mixture of ethyl acetate layer (4) and aqueous layer (5) was adjusted to 8.7 with 48% aqueous sodium hydroxide solution at room temperature with stirring to give an aqueous reverse extract (50 ml). The extract contained 4.4 g of pravastatin sodium (containing 84.88% pure pravastatin sodium), the purity of which was determined by HPLC under conditions referred to above as A.

### (2) Purification by salting-out technique

The aqueous reverse extract obtained above was concentrated under reduced pressure until the volume halved. The mixture was then adjusted to pH 12 with 48% aqueous sodium hydroxide solution. The resulting mixture was treated with sodium chloride according to the procedure of Example 1 to afford crystals containing 99.54 % pure pravastatin sodium obtained by salting-out technique, the purity of which was determined by HPLC under the conditions referred to as A. The crystals were recrystallized by a conventional procedure, washed and dried at 40°C in vacuo to give crystals (2.14 g) of pravastatin sodium containing 99.85% pure pravastatin sodium, the purity of which was determined by HPLC under the conditions referred to above as B.
In addition, instead of the base-decomposition (using an inorganic base) of the concentrated cultured broth under the condition mentioned hereinbefore, the solution (60 ml), which was obtained by evaporation of 20% of the water of the aqueous reverse extract mentioned above, was cooled to 0°C. Four equivalents of sodium hydroxide in aqueous solution were added to the concentrated solution and the resulting mixture (about pH 14) was stirred at 0°C for 30 hours (base-decomposition using an inorganic base) to afford purer pravastatin sodium than that obtained above.

### Example 8

After microbial conversion the cultured broth (10 L) containing pravastatin was adjusted to pH 12 with sodium hydroxide and the solution was stirred for 30 minutes at 50°C. After cooling the resulting mixture to room temperature, Celite 545 (trademark) (500 g, product of Celite Corp.) was added thereto as a filtration aid and the mixture was filtered. Separated mycelium was re-suspended in water (3 L). The suspension was filtered. The filtrates were combined to give a filtrate (10 L). The pH of the filtrate was adjusted to 5.7 with 25% aqueous sulfuric acid solution. Pravastatin was extracted with propyl acetate (5 L) from the aqueous solution with stirring. The pH of the separated water layer was adjusted to 5.7 with 75% aqueous sulfuric acid solution and this solution was re-extracted with propyl acetate (5 L) with stirring. The propyl acetate layers were combined and this solution was washed with saturated aqueous sodium chloride solution with stirring. The upper layer (8 L) was separated and water (1 L) was added thereto. The pH of the resulting mixture was adjusted to 9.5 with 25% aqueous sodium hydroxide solution and the water layer (1 L) containing pravastatin sodium was separated. To the aqueous solution was added ethanol (350 ml) and the pH of the mixture was adjusted to 3.0 with phosphoric acid. The resulting mixture was stirred at 50°C for 10 minutes. The pH of this mixture was adjusted to 12 with 25% aqueous sodium hydroxide solution and the resulting mixture was stirred at 50°C for 30 minutes. The solution was concentrated under reduced pressure using a rotary evaporator until the volume become 800 ml. The pH of the concentrated solution was adjusted to 12 with 48% aqueous sodium hydroxide solution and the resulting mixture was treated with sodium chloride according to Example 1 to afford crystals (60 g) containing 99.7% pure pravastatin sodium, the purity of which was determined by HPLC under the conditions referred to as C. The crystals were then recrystallized by a conventional procedure, washed and dried at 40°C in vacuo to afford crystals (55 g) containing 99.85% pure pravastatin sodium, the purity of which was determined by HPLC under the conditions referred to as C.

## Claims

1. A method for purification of pravastatin or a pharmacologically acceptable salt thereof using a salting-out technique.

2. A method according to claim 1 wherein the salt employed in the salting-out technique is an alkali metal salt, an alkaline earth metal salt or an ammonium salt.

3. A method according to claim 1 wherein the salt employed in the salting-out technique is an alkali metal salt or an ammonium salt.

4. A method according to claim 1 wherein the salt employed in the salting-out technique is sodium chloride, ammonium sulfate, ammonium acetate or ammonium nitrate.

5. A method according to claim 1 wherein the salt employed in the salting-out technique is sodium chloride.

6. A method according to any one of claims 1 to 5 which further comprises decomposition of impurities by an inorganic base.

7. A method according to any one of claims 1 to 6 which further comprises decomposition of impurities by an inorganic acid.

8. A method according to claim 7 wherein the inorganic acid is phosphoric acid or sulfuric acid.

9. A method according to claim 7 wherein the inorganic acid is phosphoric acid.

10. A method according to any one of claims 7 to 9 wherein the pH of the solution in the inorganic acid decomposition process is in the range from 2 to 5.

11. A method for purification of pravastatin sodium according to any one of claims 1 to 10.
